# EUROPÄISCHE PATENTSCHRIFT

(11) **EP 3 710 833 B1**
(45) Veröffentlichungstag und Bekanntmachung des Hinweises auf die Patenterteilung: **19.02.2025**
(21) Anmeldenummer: 18807229.2
(22) Anmeldetag: 12.11.2018
(51) Int. Cl.: G01N 33/50, C12M 3/00, C12M 1/00, C12M 1/42, C12M 1/34

(54) **EINWEG-GEHÄUSE MIT ZELLEN UND/ODER CO-KULTUREN ZUR IN-SITU-TESTUNG**
SINGLE-USE HOUSING CONTAINING CELLS AND/OR CO-CULTURES FOR IN SITU TESTING
BOÎTIER JETABLE COMPRENANT DES CULTURES DE CELLULES ET/OU DES CO-CULTURES POUR UN ESSAI IN SITU

(30) Priorität: 13.11.2017 DE 102017126629
(43) Veröffentlichungstag der Anmeldung: 23.09.2020
(73) Patentinhaber: Fachhochschule Südwestfalen, 58636 Iserlohn (DE)
(72) Erfinder: HENNES, Kilian, 58638 Iserlohn (DE)
(74) Vertreter: Michalski Hüttermann & Partner Patentanwälte mbB
(86) Internationale Anmeldenummer: PCT/EP2018/080969
(87) Internationale Veröffentlichungsnummer: WO 2019/092252

(56) Entgegenhaltungen:
- WO-A1-2013/059946
- WO-A2-2010/009307
- DE-A1- 102007 007 718
- DONGEUN HUH ET AL: "Reconstituting Organ-Level Lung Functions on a Chip", SCIENCE, vol. 328, no. 5986, 25 June 2010 (2010-06-25), pages 1662 - 1668, XP055543104, DOI: 10.1126/science.1188302

## Beschreibung

Die Erfindung betrifft das Gebiet der biologischen Toxizitäts- und Wirksamkeitstests insbesondere Gehäuse, Halterungen beinhaltend die Gehäuse, Vorrichtungen beinhaltend Halterung und Gehäuse, sowie Verfahren unter Verwendung des Gehäuses.

Biologische Reinheitsprüfungen sind bei der Herstellung von Kosmetika, Medizinprodukten und Arzneimitteln oder in Aufenthaltsbereichen des Menschen erforderlich. So sind beispielsweise Prüfungen anhand von Hautmodellen nach der neuen Kosmetik- oder REACH-Verordnung erforderlich. Hautmodelle aus kleinen Kunststoffbechern mit porösem Boden im Durchmesser von 0,5 bis 3 cm Durchmesser sind nach den Vorgaben der OECD kommerziell erhältlich. Sie werden lose in Kulturgefäßen transportiert und müssen unter aseptischen Bedingungen im Zellbiologielabor mit extern gewonnenem und aufbereitetem Probenmaterial befüllt werden. Diese Hautmodelle werden anschließend in Spezialbrutschränken weiterkultiviert und nach ein bis zwei Tagen mit optischen Lesegeräten für Laborkulturgefäße oder histologisch ausgewertet. Eine Verwendung derartiger Hautmodelle ist daher an ein zellbiologisches Speziallabor gebunden. Entsprechende Tests können daher bisher nicht direkt am Produktionsort durchgeführt werden können.

Des Weiteren sind Konzepte zur biologischen Luftuntersuchung nach dem Stand der Technik wie das Lung-on-a-Chip-Konzept bekannt. Die DE 10 2006 020 494 A1 offenbart ein künstliches Lungensystem, das zur Herstellung eines Lungenmodellsystems wie auch als Ersatz für Tiermodellsysteme bei toxikologischen Untersuchungen geeignet ist. Auch die US 2016/0145554 A1 offenbart ein Organ-on-chip System, das zur Kultivierung von Lungen- wie auch Hautgewebe geeignet ist. Lung-on-a-Chip-Vorrichtungen werden als miniaturisierte Lungenmodelle in Forschungslaboratorien eingesetzt, um die Grundlagen der Effekte von giftigen Substanzen auf die Lunge zu studieren. Sie sind optimiert für die Untersuchung von komplexen zellulären Wechselwirkungen unter mechanischer Belastung, die derjenigen im Lungengewebe entspricht. Sie sind jedoch nicht geeignet für Messungen außerhalb zellbiologischer Labore und insbesondere für Vor-Ort-Messungen oder für die Prüfung großer Luftvolumina. Auch ihre Herstellung ist komplex und damit nur bedingt serientauglich. Kulturbehälter zum Kultivieren und Differenzieren von Zellen und Geweben sind ebenfalls in vielfacher Form bekannt. Beispielsweise offenbart die DE 19952847 A1 einen Kulturbehälter mit geschlossenen Kammern mit einem Kultivierbereich und einem Einlass und einem Auslass für ein Kultur- und/oder Behandlungsmedium. Jedoch ist der Kulturbehälter nicht für Lungen- oder Hautspezifische Co-Kulturen verwendbar. DE 10 2007 007 718, beschreibt einen Einweg-Bioreaktor, wobei aus einer unteren Gehäusehälfte und einer oberen Gehäusehälfte, die mittels eines Foliengelenks jeweils an einer Seite miteinander verbunden sind, eine Kammer ausgebildet wird.

Der vorliegenden Erfindung lag daher die Aufgabe zu Grunde, eine Einwegvorrichtung für transportfähige Co-Kulturen, die außerhalb von biologischen Laboren zur Beprobung einsetzbar ist, bereitzustellen.

Die Aufgabe wird gelöst durch ein Einweg-Gehäuse wie beansprucht, zur Einhausung von Zellen und/oder Co-Kulturen für in-vitro-Prüfungen, mit einer Mehrzahl voneinander getrennter Kammern, welche jeweils wenigstens eine Einlassöffnung und eine Auslassöffnung für ein Kultur- und/oder Behandlungsmedium aufweisen, wobei das Gehäuse aus einem ersten Gehäuseteil und einem zweiten Gehäuseteil gebildet wird, und wobei in den Kammern in dem ersten Gehäuseteil eine für das Kultur- und/oder Behandlungsmedium permeable Trägerschicht fixiert ist, wodurch in den Kammern jeweils ein erstes und ein zweites Innenlumen ausgebildet werden.

Das erfindungsgemäße Einweg-Gehäuse ermöglicht, eine vorgefertigte Vorrichtung mit Testzellen und Nährstoffreservoiren außerhalb biologischer Labore ohne biologischtechnisches Personal zu Prüfzwecken einzusetzen. Hierdurch werden insbesondere im Rahmen von Zulassungsverfahren erforderliche Messungen an Hautmodellen im Produktionsumfeld ermöglicht. Weiter wird ermöglicht, direkt in Wohn- und Arbeitsbereichen eine Luftprobennahme und biologische Überprüfung vor Ort zu kombinieren, um diese Bereiche bezüglich einer erwünschten Unbedenklichkeit zu charakterisieren. Dadurch können Luftschadstoffe direkt und ohne Probenverfälschung am Ort der Probenahme biologisch nachgewiesen werden. Ein großer Vorteil ist ferner, dass mittels der Einwegvorrichtung in Kombination mit einer mobilen Mess- und/oder Auslese-Vorrichtung am Einsatzort auf die gerätetechnische und personelle Ausstattung eines Zellbiologielabors verzichtet werden kann. Vorteilhaft ist ferner die Serientauglichkeit insbesondere im Vergleich zu Lung-on-a-Chip-Methoden.

Durch die permeable Trägerschicht werden, insbesondere im geschlossenen Zustand, in den Kammern jeweils ein erstes und ein zweites Innenlumen ausgebildet. Hierdurch können in vorteilhafter Weise im ersten Innenlumen biochemische Bedingungen erzeugt werden, die ein Überleben oder Wiederbeleben von Zellen in und an dem Innenlumen sichern, nachdem die Einweg-Gehäuse von zellbiologischer Versorgung abgetrennt waren. Das erste Innenlumen kann als Nährstoffreservoir und das zweite Innenlumen als Prüfsubstanzkammer ausgebildet sein.

In bevorzugten Ausführungsformen weisen die beiden Gehäuseteile jeweils eine Reliefstruktur auf und sind scharnierartig verbunden, wobei die Reliefstrukturen der beiden Gehäuseteile im geschlossenen Zustand ineinander greifen, wobei sie die Kammern ausbilden. Dadurch, dass die beiden Gehäuseteile scharnierartig verbunden sind, sind sie faltbar bzw. "zusammenklappbar". Beispielsweise über einen Falz oder Doppelfalz zwischen den beiden Gehäuseteilen können diese über einen Faltmechanismus geschlossen und auch wieder geöffnet werden. Das Einweg-Gehäuse kann insbesondere als Kunststoff-Faltrelief ausgeführt sein. Die Gehäuseteile sind durch Tiefziehen aus Kunststofffolien herstellbar, beispielsweise im Blisterverfahren. Vorteilhaft ist daher auch die Herstellungsmöglichkeit des Einweg-Gehäuses, da Kunststoffreliefs durch Kunststofftiefziehverfahren kostengünstig produziert werden können. Geringe Fertigungskosten sind insbesondere für Einweg-Vorrichtungen vorteilhaft.

Die Reliefstrukturen der beiden Gehäuseteile greifen im geschlossenen Zustand ineinander, wobei sie voneinander getrennte Kammern ausbilden. Hierbei kann in beiden Gehäuseteilen jeweils ein gegenüberliegendes Kammerteil, insbesondere eine Kammerhälfte, ausgeformt sein, die im geschlossenen Zustand bzw. bei zugeklappten Kunststoffreliefs jeweils eine Kammer mit einer Trägerschicht darin ausbilden. Durch die Trägerschicht wird der Hohlraum der Kammern auch im zugeklappten Gehäuse in zwei Innenlumen unterteilt. Die einzelnen Kammern sind dabei jeweils voneinander getrennt. Die beiden Gehäuseteile können im Folgenden auch als "Ober-" und "Unterteil" bezeichnet werden, wobei der erste Gehäuseteil, in dem die Trägerschicht fixiert ist, als "Unterteil" bezeichnet wird und das hierdurch gebildete Teillumen des Kammerhohlkörpers als "unteres" Innenlumen, und entsprechend der zweite Gehäuseteil als "Oberteil" und dessen Lumen als oberes Innenlumen bezeichnet.

In bevorzugten Ausführungsformen werden die Kammern durch Passung ineinander eingreifbarer Reliefstrukturen in den beiden Gehäuseteilen verschlossen, vorzugsweise mittels Rippung oder Feder und Nut-Verbindung. Ein zugeklapptes Kunststoffrelief kann dabei durch geeignete Passung mit gegenüber gelegenen Reliefstrukturen eine gas- und/oder flüssigkeitsdichte Trennung der einzelnen Kammern zur Verfügung stellen. Es ist vorteilhaft, dass insbesondere die unteren Innenlumen, die mit Kultur- oder Nährmedien für Testzellen gefüllt sein können, während eines Transportes auslaufsicher versiegelt und mit den oberen Innenlumen abdeckbar sind. Hierbei kann das untere Innenlumen des Einweg-Gehäuses als Nährstoffreservoir zur Zellversorgung während einer Trennung der Medienversorgung von Einlass- und Auslassöffnungen dienen. Weiterhin kann durch Vorsehen einer hydrophoben Oberflächenbeschichtung im Bereich der Reliefstrukturen bzw. in der Nut-Feder-Region auch ein Schutz vor kapillaren Flüssigkeitsverlusten vorgesehen sein.

Die Trägerschicht kann nach der Ausformung eines Kunststoffgehäuses aufgesiegelt und entlang der die Kammern trennenden Nuten geschnitten werden. Alternativ können die Nuten nach Aufsiegelung der parallelen Außenkanten durch Federn in einem Siegelstempel zur Trägerschichtspannung beim vollständigen Aufsiegeln der übrigen Auflagebereiche in einem zweiten Schritt dienen. Die Trägerschicht kann aus biodegradierbarem oder nicht degradierbaren, beständigem Material oder deren Gemischen ausgebildet sein. Bevorzugte biodegradierbare Materialien sind Poly-L-Laktid, Polyhydroxybuttersäure, Polygycolsäure, Hyaluronsäure oder deren Gemische. Bevorzugte beständige Materialien sind Polyethylen, Polyamid, Polyester, Polyurethan, Polypropylen, Goretex oder deren Gemische. Vorzugsweise ist die Trägerschicht porös und damit permeabel für das Kultur- und/oder Behandlungsmedium. Die Trägerschicht kann als Filtermembran ausgebildet sein. Die Trägerschicht kann vor der Aufsiegelung mit adsorptionsfördernden Substanzen wie Collagen beschichtet werden. Hierdurch kann die spätere Ansiedelung der Zellen verbessert werden. Die scharnierartige Verbindung und die durch Passung ineinander eingreifbaren Reliefstrukturen in Ober- und Unterteil ermöglichen weiter, dass der obere Gehäuseteil über die gesamten Trägerschichtflächen auch nach einer Fixierung von Testzellen auf der Trägerschicht noch aufklappbar ist und einen Kontakt von Probenmaterial wie Luft, kosmetischen Zusammensetzungen oder ggf. in einem Medium enthaltenen Substanzen, mit der zelltragenden Trägerschicht ermöglichen. Die Trägerschicht ist vorzugsweise für Kultur- und/oder Behandlungsmedien durchlässig. Eine mediendurchlässige Trägerschicht ermöglicht eine Versorgung von auf die Trägerschicht aufgesiedelten Testzellen durch die Trägerschicht aus einem darunterliegenden Innenlumen, sowie den Transport von Stoffwechselprodukten der Testzellen in das Innenlumen zur anschließenden Detektion. Auch eine Durchlässigkeit für Luftvolumina von mehr als 10 L kann vor einem Kontakt der Testzellen mit der Trägerschicht nützlich sein, um die aus dem Luftvolumen zurückgehalten Luftpartikel anschließend zu charakterisieren.

Unter "Medium" wird im Rahmen dieser Anmeldung eine Flüssigkeit verstanden, die zur Kultur von Zellen geeignet ist und in dem Einweg-Gehäuse enthalten sein kann. Dabei handelt es sich bevorzugt um ein klassisches Zellkulturmedium wie DMEM (Dulbecco's Modified Eagle Medium), RPMI- (Roswell Park Memorial Institute) Medium, oder ein anderes, insbesondere für die Kultur humaner Zellen verwendbares Medium, das hierzu gegebenenfalls mit Zusätzen wie fötalem Kälberserum, Wachstumsfaktoren, Antibiotika oder geeigneten Substratanaloga versehen sein kann. Als Substratanaloga werden hier solche verstanden, die nach ihrer Umsetzung im verbrauchten Medium physikalisch oder elektrochemisch nachweisbar sind. Unter Medium werden auch andere, im Allgemeinen für die Zellkultur verträgliche Flüssigkeit wie PBS (Phosphate buffered saline) verstanden. Auch Lösungen etwa zum Ablösen von Zellen, wie Trypsin/EDTA werden im Rahmen dieser Erfindung als Medium bezeichnet. Ist ein Kulturmedium mit Substanzen versetzt, die nicht der Versorgung der Zellen dienen, sondern diese beeinflussen oder untersucht werden sollen, wird das Medium entsprechend nicht als Kulturmedium, sondern als Behandlungsmedium bezeichnet.

In Ausführungsformen weisen die Kammern im ersten und/oder zweiten Gehäuseteil jeweils eine Einlassöffnung und eine Auslassöffnung für ein Kultur- und/oder Behandlungsmedium auf. Die Kammern können im ersten, oder "unteren", Gehäuseteil jeweils eine verschließbare Einlassöffnung und eine verschließbare Auslassöffnung für ein Kultur- und/oder Behandlungsmedium aufweisen. Einlassöffnung und Auslassöffnung ermöglichen hierbei, dass die unteren Innenlumen mit Kultur- oder Nährmedien für die Testzellen durchströmbar sind. Vorzugsweise weisen die Kammern in beiden Gehäuseteilen jeweils eine verschließbare Einlassöffnung und eine verschließbare Auslassöffnung auf. Durch die Trägerschicht können die Kammern hierbei in ein oberes und ein unteres durchströmbares Innenlumen mit je einer verschließbaren Einlass- und einer Auslassöffnung geteilt werden. Dies ermöglicht, dass eine beidseitig von Zellen besiedelte Trägerschicht auf beiden Seiten von gleichen oder verschiedenen flüssigen Kultur- oder Behandlungsmedien oder gasförmigen Medien kontaktiert werden kann.

Die Einlassöffnungen und Auslassöffnungen sind gas- und/oder flüssigkeitsdicht verschließbar. Vorzugsweise sind die Einlassöffnungen und Auslassöffnungen hierbei durch ein Septum, insbesondere in einem Stutzen, verschließbar. Es ist insbesondere vorteilhaft, wenn die Einweg-Gehäuse während eines Transportes auslaufsicher verschließbar sind. Unter einem Septum wird hierbei eine Verschlussmembran aus einem elastischem Material verstanden, welche sich beispielsweise nach Durchstechen mit einer Kanüle selbsttätig wieder verschließt. Ein derartiges Septum kann beispielsweise durch Silikonschaum ausgebildet werden. Ein Septum wird vorzugsweise in einem Stutzen ausgebildet, der sich während der Herstellung des Kunststoffreliefs des Einweg-Gehäuses durch Tiefziehen gut herstellen lässt. Ein solcher Stutzen kann nach Entfernen eines eingestochenen Hohlkörpers alternativ oder zusätzlich mit Klebefolie verschlossen werden. Durch ein Septum in einem Stutzen lässt sich ein angespitzter Hohlkörper mit Schlauchverbindung einführen und damit ein Anschluss der Kammern des Einweg-Gehäuses an externe Gas- oder Flüssigkeitsreservoire herstellen.

In Ausführungsformen können die Kammern des Einweg-Gehäuses im zweiten Gehäuseteil, d.h. im Oberteil, eine Öffnung aufweisen. Eine solche Öffnung kann einen Luft- oder Umgebungskontakt des oberen Innenlumens ermöglichen. Dies ist beispielsweise für eine Luftbeprobung unter Verwendung eines Lungenmodells vorteilhaft.

Zur Verwendung für in-vitro-Prüfungen kann die Trägerschicht mit Zellen besiedelt werden. Das durch Septen verschließbare Einweg-Gehäuse ermöglicht, dass Zellen unter sterilen und pyrogenfreien Bedingungen, beispielsweise eines Zellkultur-Labors, aufgesiedelt werden können, und die vorkultivierten Zellen im Einweg-Gehäuse transportfähig vorliegen. Vorkultivierte Zellen können als Co-Kulter, beispielsweise in Form eines Haut- oder Lungenmodells, im Einweg-Gehäuse fixiert und für sofortige in-vitro-Prüfungen verwendbar versendet werden. Desweiteren können Zellen in dem unteren Innenlumen gefriergetrocknet und nach einer Lagerung bei Raumtemperatur für den anschließenden Testeinsatz mit Medium rekonstituiert werden.

**In** dem Einweg-Gehäuse ist eine Mehrzahl von Kammern voneinander getrennt ausgebildet, wodurch parallele Versuche einschließlich Kontrollen möglich sind. Ein Vorsehen von vier Kammern ermöglicht beispielsweise ein FDA-kompatibles Beproben im 2+2-Format, mit gleichzeitig zwei Prüf- und zwei Kontrollansätzen aus einer Probe. Weiterhin können dem Einweg-Gehäuse auslesbare Information über die Zellen beigegeben werden. So kann ein Code oder Barcode aufgeprägt oder aufgeklebt werden, wodurch Daten chargenweise verwendeter Zellen auslesbar und bei der Ergebnisberechnung nutzbar sind.

Abhängig von der beabsichtigten Art der in-vitro-Prüfungen kann die Trägerschicht einseitig oder zweiseitig mit Zellen besiedelt sein. Eine Besiedelung der Trägerschicht durch Testzellen unter aseptischen Bedingungen ist bevorzugt. Insbesondere ist das Einweg-Gehäuse auch für in-vitro-Prüfungen unter Verwendungen von Co-Kulturen verwendbar. Hierfür ist die für das Kultur- und/oder Behandlungsmedium permeable Trägerschicht in Ausführungsformen auf der dem ersten, oder "unteren", Innenlumen zugewandten Seite mit ersten Zellen besiedelt und auf der dem zweiten, oder "oberen", Innenlumen zugewandten Seite mit zweiten und optional weiterhin dritten Zellen besiedelt. Die Zellen können insbesondere humane Zellen sein. Diese können durch die zweiseitige Besiedlung die organotypischen Zellschichten im gesunden oder kranken Organismus modellartig abbilden.

Die ersten und zweiten Zellen sind vorzugsweise Zelltypen wie sie in Co-Kultur-Modellen verwendet werden, beispielsweise Fibroblasten und Keratinozyten in Haut-Modellen oder Endothelzellen und Lungenepithelzellen in Lungen-Modellen. Diese Modelle können mit oder ohne zusätzliche Monozyten, die die dritten Zellen bilden, ausgebildet sein. Zellen wie Epithelzellen, Fibroblasten oder Monozyten können nach bekannten Verfahren als Primärkulturen gewonnen werden oder sind kommerziell erhältlich. **In** bevorzugten Ausführungsformen ist die Trägerschicht auf der dem ersten, oder "unteren", Innenlumen zugewandten Seite mit Fibroblasten und auf der dem zweiten, oder "oberen", Innenlumen zugewandten Seite mit Keratinozyten besiedelt. In Anwendungsbeispielen, in dem an der unteren Trägerseite Fibroblasten und an der oberen, luftzugewandten Seite der Trägerschicht Keratinozyten angezüchtet werden, entstehen in dem Einweg-Gehäuse in mehreren voneinander getrennten Kammern dreidimensionale trägerstabilisierte Hautmodelle, die beispielsweise zur Prüfung von Kosmetika oder Arzneimitteln eingesetzt werden können. Hierzu können mit Fibroblasten und Keratinozyten besiedelten Einweg-Gehäuse geöffnet und die zu prüfenden Kosmetika auf einen Teil der Hautmodelle aufgetragen werden, während die Hautmodelle in den verbleibenden Kammern parallel als Kontrolle dienen können. Hierbei können Stoffwechselprodukte der zweiten Zellen oder insbesondere der ersten Zellen nachgewiesen und anhand der Unterschiede der an den Hautmodellen gemessenen Signale die Effekte der Kosmetika charakterisiert werden. Durch zusätzliche Co-Kultivierung von dritten Zellen wie Monozyten können auch immunologische Effekte untersucht werden.

In ebenfalls bevorzugten Ausführungsformen ist die Trägerschicht auf der dem ersten, oder "unteren", Innenlumen zugewandten Seite mit Endothelzellen und auf der dem zweiten, oder "oberen", Innenlumen zugewandten Seite mit Lungenepithelzellen besiedelt. Hierbei kann das erste Innenlumen mit Nährmedium gefüllt oder durchströmt werden. In einem solchen Anwendungsbeispiel werden auf der flüssigkeitszugewandten Seite der Trägerschicht Endothelzellen und an der luftzugewandten Seite Lungenepithelzellen angesiedelt. Zusätzlich können den Endo- oder Epithelzellen auch Monozyten beigemengt werden, um Zytokinbedingte Wechselwirkungen zu fördern. Über die Einlass- und Auslassöffnungen des oberen Innenlumens kann ein geregeltes Vakuum angelegt werden, so dass mit einem definiertem und gegebenenfalls intermittierendem Luftvolumenstrom über die Öffnungen im oberen Gehäuseteil Umgebungsluft an den Testzellen vorbeigeleitet werden kann. Ein kontinuierlicher Nährstoffstrom durch das untere Innenlumen kann intermittierend mit Druckschwankungen überlagert werden, welche die Druckschwankungen bei der Lungenatmung simulieren. Hierzu kann das Einweg-Gehäuse an ein geeignetes, vorzugsweise mobiles Test-Gerät angeschlossen werden. Derart kann das Einweg-Gehäuse als mehrkammeriges Lungenmodell genutzt werden, um die Luft in Verkehrsmitteln, in schadstoffbelasteten Innenräumen, an Feinstaubbelasteten Verkehrspunkten oder an Arbeitsplätzen zu untersuchen. Durch Anschluss einer geregelten Piezo-Vernebelung pyrogenfreien Wassers an die Einlassöffnungen und den Verschnitt von Luftprobe und Feuchtenebel kann erreicht werden, dass die Epithelzellen während der Prüfung keine Trocknungsschäden erleiden.

Zur Durchführung von in-vitro-Prüfungen ist es bevorzugt, die transportfähigen Gehäuse mit vorkultivierten Zellen an eine mobile Mess- und Regeltechnik anzuschließen. Ein weiterer Gegenstand betrifft daher eine Halterung für ein erfindungsgemäßes Einweg-Gehäuse, wobei die Halterung je ein negatives Relief für die beiden Gehäuseteile ausbildet, wodurch das Einweg-Gehäuse flächig einspannt wird. In Ausführungsformen können in der Halterung Kanülen angebracht, beispielsweise eingeschraubt, sein, derart, dass die Kanülen die Septen beim Einspannen durchstechen, ohne die Trägerschicht zu berühren, wobei die Kanülen vorzugsweise mit Pumpen in einer Versorgungseinheit verbunden sind, so dass Behandlungsmedium oder Reaktionsflüssigkeiten und/oder Gase durch die Kammern geführt werden können. Das Zuführen und Abführen der Reaktionsflüssigkeiten und/oder Gase kann beispielsweise durch eine geeignete Steuerungssoftware vorgesehen werden. Ein weiterer Gegenstand betrifft eine Vorrichtung zur Analyse von Substanzen, umfassend ein erfindungsgemäßes Einweg-Gehäuse und eine erfindungsgemäße Halterung für das Einweg-Gehäuse. Vorzugsweise umfasst diese weiterhin geeignete Steuerungs-, Mess-, Eingabe- und Anzeigeelektronik. Abhängig von der Ausführung können in der Halterung oder der Versorgungseinheit an Reaktionsflüssigkeiten, die aus den Kammern abgeführt wurden, biochemische oder physikalische Messungen zur Bestimmung von Stoffwechselprodukten der ersten Zellen nach dem Kontakt der zweiten Zellen mit einer zu untersuchenden Substanz bzw. zur Charakterisierung der Reaktion der Zellen auf der Trägerschicht auf die Substanzen vorgenommen werden. Des Weiteren können in der Versorgungseinheit auch Reservoire für Medien und Reagenzien, sowie Schlauch- und Pumpensysteme enthalten sein.

Das Einweg-Gehäuse ist insbesondere für in-vitro-Prüfungen wie Reinheitsprüfungen, Biokompatibilitätstests, Toxizitätstests oder Wirksamkeitstests verwendbar. Gegenstand der Erfindung ist daher auch die Verwendung des Einweg-Gehäuses für in-vitro-Prüfungen, sowie ein Verfahren zum Nachweis von Substanzen unter Verwendung des Einweg-Gehäuses. Derartige Verfahren zur biologischen Charakterisierung von Proben können in vorteilhafter Weise durch Inkubation mit Testzellen in dem Einweg-Gehäuse vorgenommen werden. Eine Charakterisierung der Reaktion der Zellen kann durch die Messung von Stoffwechselprodukten der Testzellen erfolgt, welche molekularbiologisch, immunologisch, elektrochemisch oder optisch in den zellumgebenden Medien gemessen werden können.

Ein weiterer Aspekt der Erfindung betrifft ein Verfahren zum Nachweis von Substanzen, umfassend die Schritte:
a) Bereitstellen eines erfindungsgemäßen Einweg-Gehäuses und optional einer erfindungsgemäßen Halterung für das Einweg-Gehäuse,
b) Beladung des Einweg-Gehäuses mit ersten, zweiten und/oder dritten Zellen,
c) optional Lagerung und/oder Transport des Einweg-Gehäuses,
d) Inkontaktbringen der ersten, zweiten und/oder dritten Zellen mit der zu untersuchenden Substanz, und
e) Bestimmung von Stoffwechselprodukten der ersten, zweiten und/oder dritten Zellen nach dem Kontakt der ersten, zweiten und/oder dritten Zellen mit der zu untersuchenden Substanz.

Das Einweg-Gehäuses kann vorzugsweise mit ersten und zweiten Zellen beladen werden, sowie optional mit dritten Zellen. In diesen Ausführungsformen werden die zweiten und/oder dritten Zellen mit der zu untersuchenden Substanz in Kontakt gebracht, und die Stoffwechselprodukte der ersten, zweiten und/oder dritten Zellen nach dem Kontakt der zweiten und/oder dritten Zellen mit der Substanz bestimmt. Das Einweg-Gehäuses kann alternativ nur mit Monozyten beladen werden.

Das Einweg-Gehäuse kann vor der Verwendung gelagert und/oder zum Einsatzort transortiert werden.

Das Nachweisverfahren wird unter Verwendung eines oben beschriebenen Einweg-Gehäuses durchgeführt. Das Verfahren kann daher insbesondere ein Verfahren zur biologischen Probencharakterisierung mittels matrixgebundenen Zellen in durchströmten Kunststoff-Faltreliefs sein. Die zu untersuchende Substanz kann eine feste, flüssigförmige oder gasförmige Substanz sein. Die Bestimmung von Stoffwechselprodukten kann molekularbiologisch, immunologisch, elektrochemisch oder optisch in den zellumgebenden Medien erfolgen. Die Bestimmung kann beispielsweise photometrisch oder mittels IRspektroskopischen Messungen erfolgen.

Vorzugsweise erfolgt die Bestimmung mittels elektrochemischer Messungen. In bevorzugten Ausführungsformen erfolgt die Bestimmung von Stoffwechselprodukten nach dem Prinzip der elektrochemischen Dreielektrodenmessanordnung, wobei die Trägerschicht als elektrisch leitende Arbeitselektrode ausgebildet ist und Gegen- und Bezugselektrode durch die Septen in das untere Innenlumen eingeführt werden oder als mittels Siebdruck aufgebrachte elektrisch leitende Bahnen dargestellt werden. Hierfür kann eine Verbindung der mittels Siebdruck aufgebrachten Elektroden an die Außenseite des Einweg-Gehäuses mittels Durchkontaktierung der Gehäuseteile ermöglicht werden. Weiter kann die Arbeitselektrode aus redox-reaktivem Kunststoff gebildet oder mit diesem beschichtet sein. Vorzugsweise sind an der redox-reaktiven Arbeitselektrode spezifische Oxidoreduktasen zum Nachweis von Stoffwechselprodukten der Zellen immobilisiert.

Vorteilhaft ist insbesondere, dass beispielsweise Hautmodelle nicht histologisch oder nach Lyse der Zellen biochemisch ausgewertet werden müssen, sondern am intakten Modell elektrochemisch ausgelesen werden können. Hierfür können alternativ nach dem Prinzip der elektrochemischen Dreielektrodenmessanordnung mit der Trägerschicht ein oder zwei Elektroden für potentiometrische Messungen verbunden sein. Hierfür kann die Arbeitselektrode durch Beschichtung der Trägerschicht mit redox-reaktiven Materialien hergestellt werden. Weiterhin kann eine Verbindung der Elektroden an die Außenseite des Einweg-Gehäuses mittels Durchkontaktierung der Siegelfläche der Trägerschicht ermöglicht werden.

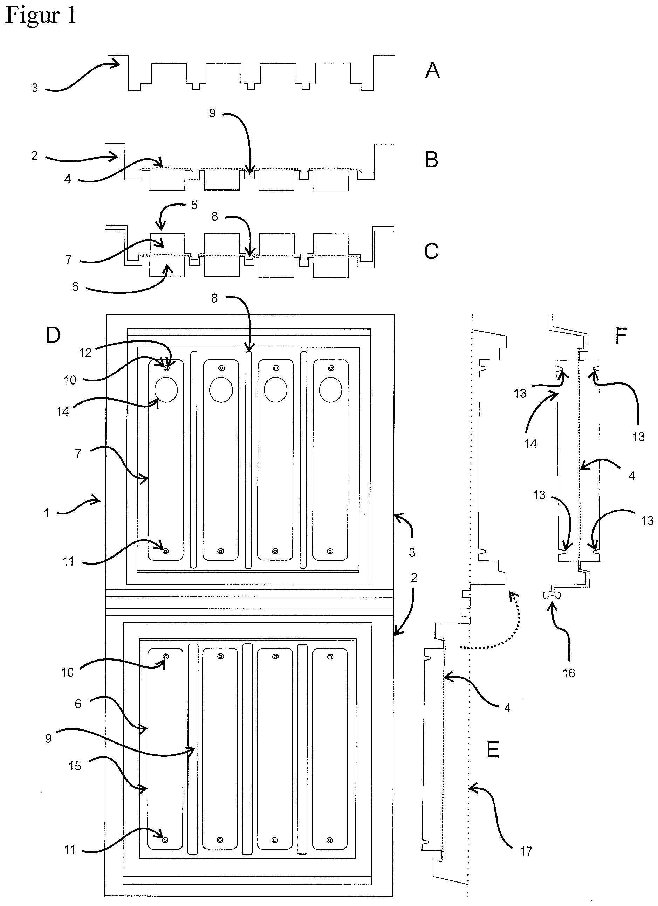

Nachfolgend wird die Erfindung anhand von Zeichnungen und Ausführungsbeispielen weiter erläutert. Darin zeigt die
- Fig. 1: eine schematische Darstellung eines Einweg-Gehäuses im Querschnitt in Fig. 1A, 1B und 1C, in Aufsicht in Fig. 1D, sowie im Längsschnitt in Fig. 1E und 1F.

Die Figuren 1A und 1B zeigen im Querschnitt einen zweiten oder oberen Gehäuseteil 3 sowie einen ersten oder unteren Gehäuseteil 2, die ein Einweg-Gehäuse 1 bilden. Die Gehäuseteile 2 und 3 weisen jeweils eine Reliefstruktur auf. Die Reliefstrukturen der beiden Gehäuseteile 2, 3 greifen im in Fig. 1C gezeigten geschlossenen oder zugeklappten Zustand ineinander, wobei vier voneinander getrennte Kammern 5 ausgebildet werden. Fig. 1B zeigt, dass im ersten Gehäuseteil 2 in den Kammern 5 eine für ein Kultur- und/oder Behandlungsmedium permeable Trägerschicht 4 fixiert ist. Hierdurch werden in den Kammern 5 jeweils ein erstes oder unteres Innenlumen 6 und ein zweites oder oberes Innenlumen 7 ausgebildet. Die Fig. 1D zeigt das Einweg-Gehäuse 1 mit dem zweiten oder oberen Gehäuseteil 3 sowie dem ersten oder unteren Gehäuseteil 2 und den vier Kammern 5 in Aufsicht. Durch Zusammenfalten des Einweg-Gehäuse 1 können die drei Federn 8 in die passgenau gefertigten Nute 9, deren Reliefstrukturen 8 und 9 im Querschnitt in Fig. 1A und 1B gezeigt sind, einschnappen und damit wie im Querschnitt in Fig. 1C ersichtlich vier Kammern 5 ausbilden und verschließen. Die Kammern 5 weisen im oberen und im unteren Gehäuseteil 3 und 2 jeweils eine Einlassöffnung 10 und eine Auslassöffnung 11 für ein Kultur- und/oder Behandlungsmedium auf. Einlassöffnungen 10 und Auslassöffnungen 11 sind mit einem Septum 12 verschlossen. Ein derartiges Septum kann beispielsweise durch Silikonschaum gebildet werden. In Fig. 1E sind im Längsschnitt ausgeformte Stutzen 13 gezeigt, in welchen die Septen 12 angebracht sind.

Das Einweg-Gehäuse 1 kann im Blisterverfahren aus Kunststofffolien tiefgezogen werden. In der Fig. 1E ist ein Längsschnitt eines Einweg-Gehäuses zu sehen, wie es nach dem Tiefziehvorgang noch im Relief sitzt. Eine Hälfte des Gehäuses wird in Fräsungen unterhalb der Aluminiumwerkzeugoberfläche 17 gezogen, während die andere Hälfte durch auf das Werkzeug aufgesetzte Formteile beim Tiefziehprozess gebildet wird. Nach der Ausformung erfolgt die Aufsiegelung einer Trägerschicht 4 in der Größe des Umrisses der in der Aufsicht Fig. 1D gezeigten Siegelfläche 15. Die Trägerschicht kann vor oder nach der Aufsiegelung mit adsorptionsfördernden Substanzen wie Collagen beschichtet werden. Nach der Aufsiegelung der Trägerschicht 4 wird diese entlang den drei kammertrennenden Nuten 9 in vier Teile geschnitten. Nun können beim Zusammenfalten des Kunststoff-Gehäuses gemäß der Klapprichtung des gestrichelten Pfeils in Fig. 1E entlang des Doppelfalzes 16 die drei Federn 8 in die passgenau gefertigten Nute 9 einschnappen und damit wie im Querschnitt der Fig. 1C ersichtlich vier Kammern 5 mit oberen Innenlumen 7 und unteren Innenlumen 6 ausbilden, die durch die vier Trägerschichten 4 getrennt werden.

Zur Besiedelung der Trägerschicht 4 mit Zellen können über die Einstichstutzen 12 bei Entlüftung an den Einstichstutzen 11 die ersten Innenlumen 6 mit einem Zellkulturmedium befüllt werden, in welchem lebende Zellen wie Fibroblasten beziehungsweise Endothelzellen enthalten sind. Diese Zellen können als Reaktion auf Umweltreize charakteristische Substanzen in die Umgebung abgeben. Das Einweg-Gehäuse 1 wird zur Besiedlung der der Trägerschicht 4 auf der dem ersten Innenlumen 6 zugewandten Seite umgedreht, wodurch das Zellen enthaltende Kulturmedium auf der Trägerschicht steht. Die Zellen werden bei einer anschließenden ersten Inkubation unter Kulturbedingungen nach dem Stand der Technik auf der dem Innenlumen zugewandten Seite der Trägerschicht 4 sedimentieren und anhaften. Während der Zellkulturinkubation kann in den vier Kammern 4 ein Medienwechsel über die Einlassöffnungen 10 und Auslassöffnungen 11 erfolgen. Wenn auf der dem ersten Innenlumen 6 zugewandten Seite der Trägerschicht eine mehrlagige Zellschicht entstanden ist, wird das zusammengeklappte Gehäuse 1 wieder geöffnet und mit der besiedelten Trägerseite nach unten positioniert.

In einem nachfolgenden Schritt kann die dem zweiten Innenlumen 7 zugewandten Seite der Trägerschicht 4 mit zweiten Zellen besiedelt werden. Hierzu kann das geöffnete Gehäuse bis zu einem Niveau oberhalb der Trägerschicht mit einem Zellkulturmedium befüllt werden, in welchem zweite lebende Zellen suspendiert sind, insbesondere solche, die im Ursprungsorganismus charakteristisch für einen Kontakt mit der Luft sind, wie Keratinozyten, Epithelzellen oder Monozyten. Diese Zellen können später durch Umweltreize stimuliert charakteristische Substanzen in die Umgebung abgeben. Die Zellen sedimentieren bei einer anschließenden zweiten Zellkulturinkubation gegenüber der zuvor gebildeten mehrlagigen ersten Zellschicht auf die andere Seite der Trägerschicht. Die Zellen befinden sich damit an der Trägerschichtseite, die im zusammengeklappten Zustand an die zweiten oder oberen Innenlumen 7 angrenzt. Zum Schutz der Zellen während der zweiten Inkubation kann das Gehäuse mit einer Abdeckung verschlossen werden. Die ersten Zellen werden während der zweiten Inkubation ebenfalls weiter mit Medium versorgt, wobei das Kulturmedium wird in den unteren Innenlumen 6 über die Einlass- und Auslassöffnungen 10 und 11 möglichst blasenfrei erneuert wird. Sobald sich eine zweite hinreichend dicke Zellschicht auf der Oberfläche der Trägerschichten 4 gebildet hat, wird das Kulturmedium über den Trägerschichten entnommen und das Kunststoff-Gehäuse 1 wird wie im Querschnitt C dargestellt wieder verschlossen. Anschließend wird die Kulturflüssigkeit in den unteren Innenlumen 6 erneut gewechselt. Durch die vier Öffnungen 14 im zweiten bzw. oberen Gehäuseteil 3 zu den vier Kammern 5 hat die mehrlagige trägerstabilisierte zweite Testzellschicht nun Luftkontakt. Anschließend kann die Versorgung mit Kulturmedium des zusammengeklappten Kunststoff-Gehäuses 1 abgetrennt werden. Die Septen 12 können die Kammern 4 dicht verschließen. Zusätzlich können und die Stutzen 13 mit Klebefolie versiegelt werden.

### Bestimmung von Luftschadstoffen

Das mit Co-Kulturen aus ersten, zweiten und dritten Zellen besiedelte Einweg-Gehäuse kann in dieser Form steril verpackt und beispielsweise per temperiertem Postversand zum Einsatzort der in-vitro-Prüfungen gebracht werden. Am Einsatzort kann das Einweg-Gehäuse an eine Halterung angeschlossen werden. Die Halterung weist geeigneter Weise je ein negatives Relief für die beiden Gehäuseteile 2 und 3 auf, wodurch das Einweg-Gehäuse 1 flächig einspannt werden kann. Die Halterung kann ein mobiles Gerät der Mess- und Regeltechnik sein, welches die Fließrate und Temperatur des dann kontinuierlich erneuerbaren Kulturmediums steuert und die Temperatur in den vier Kammern für die Testzellen optimiert. Hierzu können die Septen 12 in Einlass- und Auslassöffnungen 10 und 11 mittels Kanülen durchstochen werden. In der Halterung können hierzu Kanülen derart angebracht sein, dass diese die Septen beim Einspannen durchstechen ohne die Trägerschicht 4 zu berühren. Die Kanülen können insbesondere mit Pumpen in einer Versorgungseinheit verbunden sein, so dass Behandlungsmedien und/oder Gase für in-vitro-Prüfungen durch die Kammern 5 geführt werden können.

Eine solche Vorrichtung umfassend Einweg-Gehäuse und Halterung ist für in-vitro-Prüfungen bzw. biologische Tests zur Analyse von Substanzen am Einsatzort ausgerüstet. Hierzu werden die zweiten Zellen wie Epithelzellen und die dritten Zellen wie Monozyten mit der zu untersuchenden Substanz in Kontakt gebracht. Hierzu kann beispielsweise ein Luftstrom durch die Öffnungen 14 im zweiten bzw. oberen Gehäuseteil 3 über die Schicht der Epithelzellen geleitet werden. Epithelzellen und Monozyten können durch die Umweltreize stimuliert charakteristische Substanzen in die Umgebung abgeben, auf die die Endothelzellen reagieren und ihrerseits charakteristische Substanzen in das umgebende Medium abgeben können. Stoffwechselprodukte der Endothelzellen können daraufhin bestimmt werden. Hierzu kann das mobile Gerät über eine geeignete elektronische oder optische Sensorik verfügen, mit der Stoffwechselprodukte der zweiten Testzellen direkt oder indirekt nachgewiesen werden können. Dabei können biochemische, molekularbiologische, immunologische oder elektrochemische Reaktionen sowohl an den Zellschichten als auch im Kulturmedium zur Erzeugung der elektronischen oder optischen Signale beteiligt sein.

Zur Qualitätssicherung der Prüfungen können zwei der vier Kammern als Positivkontrollen mit den zu testenden Analyten beaufschlagt werden und zwei Kammern zur eigentlichen Messung dienen. Das mobile Gerät der Mess- und Regeltechnik kann weiter dazu dienen, von den gemessenen Signalen Mittelwerte zu errechnen und die Validität der Messungen anhand der Positivkontrollen zu überprüfen. Das mobile Gerät kann ferner über eine Ergebnisanzeige, einen Internetanschluss und/oder eine Speichermöglichkeit für Testergebnisse und Chargendaten des Einweg-Gehäuses verfügen.

### Beispiel 1

### Herstellung der Einweg-Gehäuse

Zur Herstellung des in Fig. 1 dargestellten Einweg-Gehäuses wurde eine farblos transparente PET-GAG Folie mit einer Stärke von 0,4 mm (PET-GAG 340, glänzend, 400 x 0,4 mm, Folien Werk Wolfen) verwendet und die beiden Gehäuseteile wurden als Blister-Kassette im Tiefziehprozess bei 540°C auf einer Tiefziehanlage SKA3550T (Zappe GmbH) hergestellt.

Eine Versapore-Membran (Versapore, 100W/WA 10'x3M, Porengröße 1,2 µm, Pall Corporation) wurde auf Maße von 1,6 × 8,5 cm zugeschnitten und zur Dekontamination für 72 Stunden in Anosteralyt^{®} (1:3 verdünnt, WABOSAN Arzneimittelvertriebs GmbH) gelagert. Vor dem Aufsiegeln wurde die Versapore-Membran anschließend für 2 Minuten auf Küchenpapier gelegt. Silikon-Backpapier wurde als Unterlage um die Kavitäten der Blister-Kassetten gelegt. Das Siegeln der Membran erfolgt auf einer Blister Siegelmaschine QUICK (Zappe GmbH) bei 120°C, für 20 Sekunden und bei einem Druck von 5 bar.

Zum Abdichten der Einlass- und Auslassöffnungen der Blister-Kassetten wurden 2 µl Loctite 4304 LC Kleber (Henkel) in die Stutzen gegeben und im Anschluss daran wurden die Stutzen mit Foam Cord (Coltene) aufgefüllt. Die Aushärtung erfolgt bei Raumtemperatur für 3 Stunden auf einem Schüttelinkubator (Schüttelinkubator 1000, Heidolph) bei 750 rpm.

Zur Dekontamination wurden die Blister-Kassetten abschließend bei 5×10⁻⁴ bar, 150 W, für 45 Sekunden mit 99% Ar (Argon C, 99,996 Vol.-%, Linde) und 1% O₂ (Sauerstoff C, 99,5 Vol.-%, Linde) im Mikrowellenplasma (100-E Plasma Systems, Technisch Plasma GmbH) behandelt.

### Beispiel 2

### Herstellung eines Hautmodells in einem Einweg-Gehäuse

a) Herstellung eines Kollagen Typ-I Gels
   Zur Herstellung eines Hautmodells in einem Einweg-Gehäuse wurde zunächst ein Kollagen Typ-I Gel, welches Fibroblasten enthält, hergestellt. Die Herstellung des Kollagen Typ-I Gel fand gekühlt bei 4°C statt. Die benötigten Materialien und Lösungen wurden im Kühlschrank gelagert und eine mit Wasser gefüllte eingefrorene Kunststoffbox diente als Kühlstation. In einem ersten Schritt wurde eine Lösung B mit 720 µl 10 × DMEM (Dulbecco's Modified Eagle Medium mit 3,7 g/l NaHCO₃, 4,5 g/l Glucose, 0,5% L-Glutamin, Biochrom), 360 µl 1M Hepes-Puffer (Ultra Saline A, Hepes Buffered Saline, Lonza) und 250 µl NaOH (0,7 mol/l, VWR) hergestellt, die anschließend im Kühlschrank gelagert wurde. Zur Herstellung von 1 ml Kollagen Typ-I Gel wurden 100 µl Lösung B und 800 µl Collagen G (4 mg/ml, Biochrom) in einem Zentrifugenröhrchen pipettiert und auf einem Reagenzglasschüttler (Vortexer) gemischt. Anschließend wurden 100 µl Zellsuspension von 5 × 10⁵ Zellen/ml in FKS (Fetal Bovine Serum, Biochrome) der Fibroblasten-Linien NIH-3T3 (Labor für Biomaterialien Iserlohn) oder Hs-68 (Sigma-Aldrich Prod. Nr.: 89051701) hinzugegeben und ebenfalls auf dem Reagenzglasschüttler gemischt. Das Kollagen Typ-I Gel wurde für 10 Minuten im Kühlschrank gelagert bis die entstandenen Luftblasen aus dem Gel hinaus diffundiert waren. Für vier Kavitäten eines gemäß Beispiel 1 hergestellten Blisterkassette betrug das benötigte Volumen an Kollagen Typ-I Gel 3,2 ml bei 800 µl Kollagen Typ-I Gel pro Kavität.
b) Aufbringen des Kollagen Typ-I Gels in das Einweg-Gehäuse
   Eine Silikonmaske wurde auf die Versapore-Membran gelegt. In Ausschnitte der Maske wurden 800 µl Kollagen Typ I-Gel mit Fibroblasten pipettiert. Die Blister-Kassetten mit den Gelen wurden im Schüttelinkubator zur gleichmäßigen Verteilung des Gels für 7 Minuten bei 600 rpm und 37°C geschüttelt. Anschließend wurden die Blister zur Aushärtung der Kollagen Typ-I Gele für 2 Stunden bei 37°C und 5 % CO₂ im Brutschrank (XC, Binder) gelagert.
c) Aufbringen der Keratinozyten
   Nach dem Aushärten der Kollagen Typ-I Gele wurden pro Kavität 3 ml Zellsuspension mit 5,3 × 10⁵ Zellen/ml der Keratinozyten-Zelllinie HaCaT (Labor für Biomaterialien Iserlohn) in DMEM hinzugegeben. Anschließend wurden die Einweg-Gehäuse im Brutschrank bei 37°C und 5% CO₂ für 24 Stunden inkubiert. Das Medium wurde anschließend abgesaugt und neues Medium wurde in die einzelnen Kavitäten auf der unbewachsenen Seite gegeben, damit die HaCaT-Keratinozyten luftexponiert wachsen konnten. Die Hautmodelle wurden bei 37°C und 5% CO₂ im Brutschrank für 14 Tage kultiviert, wobei alle 24 Stunden ein Wechsel des Mediums erfolgte.

### Beispiel 3

### Bestimmung der Lagerstabilität von humanen Keratinozyten mittels Nachweis der metabolischen Aktivität

In 24er Well-Zellkulturschalen (Wachstumsfläche: 1,82 × 10,32 cm²) wurden HaCaT Keratinozyten in einer Zelldichte von 4 - 10⁴ Zellen/cm² ausgesät und für 48 h bei 37°C, 5 % CO₂ im Brutschrank inkubiert. Anschließend wurden sechs der Zellkulturschalen für weitere 24 h bei 37°C, 5 % CO₂ im Brutschrank gelagert, während sechs weitere Zellkulturschalen für 24 h bei 6°C in einer nicht definierten Atmosphäre gelagert wurden. Anschließend wurden die Zellkulturschalen für weitere 24 h bei 37°C, 5 % CO₂ inkubiert, bevor ein Nachweis der metabolischen Aktivität der Zellen mittels MTT-Assay nach Hersteller-Angaben erfolgte. Die Vitalität der Zellen, die für 24 h bei 6°C gelagert wurden entsprach 9 % im Vergleich zu den im Brutschrank bei 37°C und 5 % CO₂ inkubierten Zellen, deren Vitalität zu 100% gesetzt wurde. Berücksichtigt man, dass die Zellen mitotisch aktiv waren und sich die Zellen bei 37°C somit unter optimalen Bedingungen weiter vermehren konnten. Würden postmitotische Zellen auf Lagerstabilität getestet, wird vermutet, dass die Vitalität an die der Referenzprobe bei 37°C Lagerung heranreichen würde.

Die Bestimmung der Vitalität zeigt, dass die humanen Keratinozyten für 24 h bei 6°C erfolgreich gelagert werden konnte. Dies zeigt, dass ein mit humanen Testzellen eines Hautmodells besiedeltes Einweg-Gehäuse gekühlt an einen Einsatzort außerhalb eines Zellkulturlabors verbracht werden kann, und dort für *in-vitro-*Prüfungen verwendbar ist.

### Beispiel 4

### Herstellungsprozess eines Lungenmodells in einem Einweg-Gehäuse

Zur Herstellung eines Lungenmodels wurden 2,4 ·10⁵ Zellen/cm² der Endothelzelllinie EA.HY-926 pro Kavität auf die Versapore-Membran (Wachstumsfläche 10,32 cm²) gegeben. Die Kavitäten wurden hierzu jeweils mit 3 ml Zellsuspension befüllt und anschließend im Brutschrank bei 37 °C und 5 % CO₂ für 4 h inkubiert bis die EA.HY-926 Zellen adhäriert waren. Anschließend wurden auf der anderen Seite der Membran 1,2 · 10⁵ Zellen/cm² der Epithelzellen A549 hinzugegeben. Dieses Lungenmodell im Einweg-Gehäuse wurde für zwei Tage bei 37°C und 5 % CO₂ im Brutschrank inkubiert. An Tag 3 wurden THP-1-Monozytenzellen wurden mittels Phorbol-12-myristat-13-acetat (PMA, Biochrom) zu Makrophagen differenziert. Zu THP-1 Zellen in einer Dichte mit 4 · 10⁵ Zellen/ml wurde PMA hinzugegeben, so dass sich eine Endkonzentration von 20 ng/ml PMA im Medium mit den THP-1 Zellen ergab. Die Zellen wurden anschließend bei 37 °C und 5 % CO₂ im Brutschrank für 24 Stunden inkubiert.

Die adhärierten und zu Makrophagen differenzierten THP-1 Zellen wurden danach nach Standardprotokoll (PANBiotech) vom Boden gelöst, mit der Abweichung, dass kein Trypsin zum Lösen verwendet wurde. Zum Lösen der THP-1 Zellen wurde stattdessen 0,5 mM EDTA (PANBiotech) verwendet. Die THP-1 Zellen wurden in einer Konzentration von 2,4 · 10⁵ Zellen/cm² auf die Seite der Membran mit den A549 Epithelzellen gegeben. Im Brutschrank bei 37 °C und 5 % CO₂ wurden die Lungenmodelle für weitere 4 Stunden inkubiert bis die THP-1 Zellen adhärent waren. Anschließend wurde das Medium für eine luftexponierte Kultivierung der A549 und THP-1 Zellen entfernt. An Tag 5 konnte die jeweils zu testende Substanz auf das Lungenmodell aufgebracht werden.

Insgesamt zeigen die Beispiele, dass Einweg-Gehäuse im Tiefziehverfahren als Blister-Kassette herstellbar sind und eine Trägerschicht für Testzellen aufgesiegelt werden kann. Die Einweg-Gehäuse wurden erfolgreich mit humanen Fibroblasten und Keratinozyten für ein Hautmodell sowie mit Endothel-, Epithelzellen und Monozyten für ein Lungenmodell besiedelt. Diese können temperiert an einen Einsatzort außerhalb eines Zellkulturlabors verbracht und dort für *in-vitro-*Prüfungen verwendet werden.

### Beispiel 5

### Herstellungsprozess eines gefriergetrockneten Monozyten-Aktivierungstest-Ansatzes im Einweg-Gehäuse

Zur Herstellung eines Testansatzes mit aktivierten und anschließend lyophilisierten Monozyten, der über mehrere Monate bei Raumtemperatur lagerfähig ist, wurde zunächst Einfriermedium hergestellt. Hierfür wurden 40 ml Ca²⁺- und Mg²⁺-freies PBS vorgelegt, 34,9 mg EGCG abgewogen und in dem PBS gelöst. Anschließend wurden 3,02 g Trehalose-Dihydrat (0,1 M) zugegeben und mit Ca²⁺- und Mg²⁺-freiem PBS auf 80 ml aufgefüllt. Nach dieser Vorbereitung wurden von 5 Blutspendern je 2 Heparinmonovetten gewonnen und nach 10-maligem leichten Schwenken in einem Blutbeutelsystem auf einem Kippmischer mit 30 Zyklen pro Minute gemischt. Danach wurden je 3 ml Histopaque in 30 Zentrifugenröhrchen gegeben, mit je 3 ml des gemischten Humanbluts vorsichtig überschichtet und für 30 min bei 1000g zentrifugiert. Die Monozyten wurden durch vorsichtiges Abpipettieren der entsprechenden Bande entnommen und in je 10 ml des Ca²⁺- und Mg²⁺-freien PBS gelöst, mit 50 mg AgNP-Lösung (50 µg/ml Endkonzentration) und 1 mg LPS-Lösung (1µg/ml Endkonzentration) versetzt, für 1 h bei 37 °C voraktiviert und anschließend zum Stoppen der Voraktivierung bei 4 °C für 10 min mit 180 g zentrifugiert. Die Pellets wurden anschließend in je 10 ml Ca²⁺- und Mg²⁺- freien PBS in Zentrifugenröhrchen resuspendiert. Die Zentrifugenröhrchen wurden für 10 min bei 250 g zentrifugiert und 4 Pellets wurden anschließend in je 2,5 ml Einfrierlösung resuspendiert und nach der Zellzahlbestimmung (beispielsweise mit einem CasyCounter) mit Einfrierlösung auf ca. 10⁷ Zellen/ml gebracht. Die Proben wurden mit Kanülen in Portionen zu je 0,5 ml auf den Boden des Innenlumens 6 von gemäß Beispiel 1 hergestellten Blisterkassetten mit Filtermembran injiziert und mit etwa 5 K/min auf -70 °C heruntergekühlt, z.B. mit flüssigem Stickstoff in einer geeigneten Vorrichtung. Die Blisterkassetten mit den tiefgefrorenen Monozyten wurden in einem Lyophilisator unter Vakuum (0,5 µbar) bei -70 °C für gefriergetrocknet.

Die so präparierten Blisterkassetten mit lyophilisierten Monozyten konnten zunächst zur Luftfiltration durch eine aufgesiegelte Filtermembran mittels einer handelsüblichen Luftprobenahmepumpe verwendet werden. Nach anschließender Resuspension der Monozyten und Flüssigkeitskontakt dieser Suspension mit den auf der Filtermembran zurückgehaltenen Luftpartikel, konnte deren Gehalt an pyrogenen Substanzen mittel Monozytenaktivierungstest nach Europäischem Arzneibuch 2.6.30 gemessen werden.

### Bezugszeichenliste:

- 1: Einweg-Gehäuse
- 2: erster Gehäuseteil
- 3: zweiter Gehäuseteil
- 4: Trägerschicht
- 5: Kammer
- 6: erstes Innenlumen
- 7: zweites Innenlumen
- 8: Feder
- 9: Nut
- 10: Einlassöffnung
- 11: Auslassöffnung
- 12: Septum
- 13: Stutzen
- 14: Öffnung
- 15: Siegelfläche
- 16: Doppelfalz
- 17: Aluminiumwerkzeug

## Patentansprüche

1. Einweg-Gehäuse zur Einhausung von Zellen und/oder Co-Kulturen für in-vitro-Prüfungen, mit einer Mehrzahl voneinander getrennter Kammern (5), welche jeweils wenigstens eine Einlassöffnung (10) und eine Auslassöffnung (11) für ein Kultur- und/oder Behandlungsmedium aufweisen, wobei das Gehäuse (1) aus einem ersten Gehäuseteil (2) und einem zweiten Gehäuseteil (3) gebildet wird, und wobei in den Kammern (5) in dem ersten Gehäuseteil (2) eine für das Kultur- und/oder Behandlungsmedium permeable Trägerschicht (4) fixiert ist, wodurch in den Kammern (5) jeweils ein erstes Innenlumen (6) und ein zweites Innenlumen (7) ausgebildet werden, und wobei die beiden Gehäuseteile (2, 3) jeweils eine Reliefstruktur aufweisen und scharnierartig verbunden sind, wobei die Reliefstrukturen der beiden Gehäuseteile (2, 3) im geschlossenen Zustand ineinander greifen, wobei sie die Kammern (5) ausbilden, und wobei die Einlassöffnungen (10) und Auslassöffnungen (11) gas- und/oder flüssigkeitsdicht verschließbar sind.

2. Einweg-Gehäuse nach Anspruch 1, **dadurch gekennzeichnet, dass** die Kammern (5) insbesondere durch Passung ineinander eingreifbarer Reliefstrukturen (8, 9) verschlossen werden, vorzugsweise mittels Rippung oder Feder und Nut-Verbindung (8, 9).

3. Einweg-Gehäuse nach Anspruch 1 oder 2, **dadurch gekennzeichnet, dass** die Kammern (5) vorzugsweise in beiden Gehäuseteilen (2, 3) jeweils eine verschließbare Einlassöffnung (10) und eine verschließbare Auslassöffnung (11) für ein Kultur- und/oder Behandlungsmedium aufweisen.

4. Einweg-Gehäuse nach einem der vorhergehenden Ansprüche, **dadurch gekennzeichnet, dass** die Einlassöffnungen (10) und die Auslassöffnungen (11) durch ein Septum (12), insbesondere in einem Stutzen (13), verschließbar sind.

5. Einweg-Gehäuse nach einem der vorhergehenden Ansprüche, **dadurch gekennzeichnet, dass** die für das Kultur- und/oder Behandlungsmedium permeable Trägerschicht (4) auf der dem ersten Innenlumen (6) zugewandten Seite mit ersten Zellen besiedelt ist und auf der dem zweiten Innenlumen (7) zugewandten Seite mit zweiten Zellen, und optional weiterhin dritten Zellen, besiedelt ist.

6. Einweg-Gehäuse nach Anspruch 5, **dadurch gekennzeichnet, dass** die Trägerschicht (4) auf der dem ersten Innenlumen (6) zugewandten Seite mit Fibroblasten und auf der dem zweiten Innenlumen (7) zugewandten Seite mit Keratinozyten besiedelt ist, oder auf der dem ersten Innenlumen (6) zugewandten Seite mit Endothelzellen und auf der dem zweiten Innenlumen (7) zugewandten Seite mit Lungenepithelzellen besiedelt ist.

7. Halterung beinhaltend ein Einweg-Gehäuse nach einem der Ansprüche 1 bis 6, **dadurch gekennzeichnet, dass** die Halterung je ein negatives Relief für die beiden Gehäuseteile (2, 3) ausbildet, wodurch das Einweg-Gehäuse (1) flächig einspannt wird.

8. Halterung nach Anspruch 7, **dadurch gekennzeichnet, dass** in der Halterung Kanülen angebracht sind, derart dass die Kanülen die Septen (12) beim Einspannen durchstechen ohne die Trägerschicht (4) zu berühren, wobei die Kanülen vorzugsweise mit Pumpen in einer Versorgungseinheit verbunden sind, so dass Behandlungsmedien und/oder Gase durch die Kammern (5) geführt werden können.

9. Vorrichtung zur Analyse von Substanzen, umfassend eine Halterung beinhaltend ein Einweg-Gehäuse nach einem der Ansprüche 7 oder 8.

10. Verfahren zum Nachweis von Substanzen, umfassend die Schritte:
a) Bereitstellen eines Einweg-Gehäuses nach einem der Ansprüche 1 bis 6 oder einer Halterung beinhaltend ein Einweg-Gehäuse nach einem der Ansprüche 7 oder 8,
b) Beladung des Einweg-Gehäuses mit ersten, zweiten und/oder dritten Zellen,
c) optional Lagerung und/oder Transport des Einweg-Gehäuses,
d) Inkontaktbringen der ersten, zweiten und/oder dritten Zellen mit der zu untersuchenden Substanz, und
e) Bestimmung von Stoffwechselprodukten der ersten, zweiten und/oder dritten Zellen nach dem Kontakt der ersten, zweiten und/oder dritten Zellen mit der zu untersuchenden Substanz.

11. Verfahren nach Anspruch 10, **dadurch gekennzeichnet, dass** die Bestimmung von Stoffwechselprodukten nach dem Prinzip der elektrochemischen Dreielektrodenmessanordnung erfolgt, wobei die Trägerschicht (4) als elektrisch leitende Arbeitselektrode ausgebildet ist und Gegen- und Bezugselektrode durch die Septen (12) in das untere Innenlumen (6) eingeführt werden oder als mittels Siebdruck aufgebrachte elektrisch leitende Bahnen dargestellt werden.

## Claims

1. Disposable housing for enclosing cells and/or co-cultures for *in vitro* tests, having a plurality of chambers (5) separated from one another which each have at least one inlet opening (10) and one outlet opening (11) for a culture medium and/or treatment medium, wherein the housing (1) is formed from a first housing part (2) and a second housing part (3), and a carrier layer (4) that is permeable to the culture medium and/or treatment medium is fixed in the chambers (5) in the first housing part (2), whereby a first inner lumen (6) and a second inner lumen (7) are formed in each of the chambers (5), and wherein the two housing parts (2, 3) each have a relief structure and are connected in a hinge-like manner, wherein the relief structures of the two housing parts (2, 3) interlock in the closed state, thereby forming the chambers (5), and wherein the inlet openings (10) and outlet openings (11) are closable in a gas-tight and/or liquid-tight manner.

2. The disposable housing according to claim 1, **characterised in that** the chambers (5) are closed particularly by fitting interlocking relief structures (8, 9), preferably by means of ribbing or tongue and groove connection (8, 9).

3. The disposable housing according to claim 1 or 2, **characterised in that** the chambers (5), preferably in each housing parts (2, 3), have a closable inlet opening (10) and a closable outlet opening (11) for a culture and/or treatment medium.

4. The disposable housing according to one of the preceding claims, **characterised in that** the inlet openings (10) and the outlet openings (11) are closable by a septum (12), in particular in a nozzle (13).

5. The disposable housing according to any one of the preceding claims, **characterised in that** the carrier layer (4) that is permeable to the culture medium and/or treatment medium is populated with first cells on the side facing the first inner lumen (6) and is populated with second cells, and optionally furthermore third cells, on the side facing the second inner lumen (7).

6. The disposable housing according to claim 5, **characterised in that** the carrier layer (4) is populated with fibroblasts on the side facing the first inner lumen (6) and with keratinocytes on the side facing the second inner lumen (7), or is populated with endothelial cells on the side facing the first inner lumen (6) and with lung epithelial cells on the side facing the second inner lumen (7).

7. A holder containing a disposable housing according to any one of claims 1 to 6, **characterised in that** the holder forms a negative relief for each of the two housing parts (2, 3), whereby the disposable housing (1) is clamped over its surface.

8. The holder according to claim 7, **characterised in that** cannulae are mounted in the holder in such a way that the cannulae pierce the septa (12) during clamping without touching the carrier layer (4), whereby the cannulae preferably are connected to pumps in a supply unit so that treatment media and/or gases can be guided through the chambers (5).

9. A device for analysing substances, comprising a holder according to any one of claims 7 or 8 containing a disposable housing.

10. A method for the detection of substances, comprising the steps of:
a) providing a disposable housing according to any one of claims 1 to 6 or a holder according to any one of claims 7 or 8 containing a disposable housing,
b) loading the disposable housing with first, second and/or third cells,
c) optionally storing and/or transporting the disposable housing,
d) bringing the first, second and/or third cells into contact with the substance to be analysed, and
e) determining metabolic products of the first, second and/or third cells after contact of the first, second and/or third cells with the substance to be analysed.

11. The method according to claim 10, **characterised in that** the determination of metabolic products is carried out according to the principle of the electrochemical three-electrode measuring arrangement, wherein the carrier layer (4) is designed as an electrically conductive working electrode and the counter electrode and reference electrode are introduced through the septa (12) into the lower inner lumen (6) or are represented as electrically conductive paths applied by means of screen printing.

## Revendications

1. Boîtier à usage unique destiné à loger des cellules et/ou des co-cultures destinées à des tests in vitro, ledit boîtier comportant une pluralité de chambres (5) qui sont séparées les unes des autres et qui comportent chacune au moins une ouverture d'entrée (10) et une ouverture de sortie (11) destinées à un milieu de culture et/ou de traitement, le boîtier (1) étant formé d'une première partie de boîtier (2) et d'une deuxième partie de boîtier (3), et une couche de support (4), perméable au milieu de culture et/ou de traitement, étant fixée dans les chambres (5) de la première partie de boîtier (2), ce qui permet de former une première lumière intérieure (6) et une deuxième lumière intérieure (7) dans les chambres (5), et les deux parties de boîtier (2, 3) comportant chacune une structure en relief et étant reliées à la manière d'une charnière, les structures en relief des deux parties de boîtier (2, 3) s'engageant les unes dans les autres à l'état fermé en formant les chambres (5), et les ouvertures d'entrée (10) et les ouvertures de sortie (11) pouvant être fermées de manière étanche aux gaz et/ou aux liquides.

2. Boîtier à usage unique selon la revendication 1, **caractérisé en ce que** les chambres (5) sont fermées notamment par ajustement de structures en relief (8, 9) qui peuvent s'engager les unes dans les autres, de préférence au moyen de nervures ou de liaison à rainure et languette (8, 9).

3. Boîtier à usage unique selon la revendication 1 ou 2, **caractérisé en ce que** les chambres (5) comportent chacune, de préférence dans les deux parties de boîtier (2, 3), une ouverture **d'entrée** fermable (10) et une ouverture de sortie fermable (11) destinées à un milieu de culture et/ou de traitement.

4. Boîtier à usage unique selon l'une des revendications précédentes, **caractérisé en ce que** les ouvertures d'entrée (10) et les ouvertures de sortie (11) peuvent être fermées par une cloison (12), notamment dans une tubulure (13).

5. Boîtier à usage unique selon l'une des revendications précédentes, **caractérisé en ce que** la couche de support (4), perméable au milieu de culture et/ou de traitement, est ensemencée de premières cellules du côté dirigé vers la première lumière intérieure (6), de deuxièmes cellules, et éventuellement en outre de troisièmes cellules, du côté dirigé vers la deuxième lumière intérieure (7).

6. Boîtier à usage unique selon la revendication 5, **caractérisé en ce que** la couche de support (4) est ensemencée de fibroblastes du côté dirigé vers la première lumière intérieure (6) et de kératinocytes du côté dirigé vers la deuxième lumière intérieure (7), ou de cellules endothéliales du côté dirigé vers la première lumière intérieure (6) et de cellules épithéliales pulmonaires du côté dirigé vers la deuxième lumière intérieure (7).

7. Support contenant un boîtier à usage unique selon l'une des revendications 1 à 6, **caractérisé en ce que** le support forme un relief négatif pour chacune des deux parties de boîtier (2, 3), ce qui permet de serrer le boîtier à usage unique (1) de manière sensiblement bidimensionnelle.

8. Support selon la revendication 7, **caractérisé en ce que** des canules sont fixées dans le support de manière à ce que les canules transpercent la cloison (12), lorsqu'elles sont serrées, sans toucher la couche de support (4), les canules étant de préférence reliées à des pompes dans une unité d'alimentation de telle sorte que des milieux de traitement et/ou des gaz puissent être guidés à travers les chambres (5).

9. Dispositif d'analyse de substances, comprenant un support contenant un boîtier à usage unique selon l'une des revendications 7 ou 8.

10. Procédé de détection de substances, ledit procédé comprenant les étapes suivantes :
a) fournir un boîtier à usage unique selon l'une des revendications 1 à 6 ou un support contenant un boîtier à usage unique selon l'une des revendications 7 ou 8,
b) charger le boîtier à usage unique avec des première, deuxième et/ou troisième cellules,
c) stocker et/ou transporter éventuellement le boîtier à usage unique,
d) mettre les première, deuxième et/ou troisième cellules en contact avec la substance à examiner, et
e) déterminer des métabolites des première, deuxième et/ou troisième cellules après contact des première, deuxième et/ou troisième cellules avec la substance à examiner.

11. Procédé selon la revendication 10, **caractérisé en ce que** la détermination de métabolites est effectuée selon le principe de l'ensemble de mesure électrochimique à trois électrodes, la couche de support (4) étant conçue comme une électrode de travail électriquement conductrice et des contre-électrodes et électrodes de référence étant insérées à travers les cloisons (12) dans la lumière intérieure inférieure (6) ou se présentant sous la forme de pistes électriquement conductrices appliquées par sérigraphie.
